Europäisches Patentamt

European Patent Office

Office européen des brevets

(11) Publication number: 0 208 468 B1

# EUROPEAN PATENT SPECIFICATION

(45) Date of publication of patent specification: 22.06.94

(51) Int. Cl.5: **C12N 15/52**, C12N 15/74, C07K 15/00

(21) Application number: 86304900.3

(22) Date of filing: 25.06.86

The file contains technical information submitted after the application was filed and not included in this specification

(54) PTR2030, a conjugal plasmid and derivatives thereof that confer phage resistance to group N streptococci.

(30) Priority: 25.06.85 US 748444

(43) Date of publication of application:
14.01.87 Bulletin 87/03

(45) Publication of the grant of the patent:
22.06.94 Bulletin 94/25

(84) Designated Contracting States:
BE DE FR GB NL

(56) References cited:

APPLIED ENVIRON. MICROBIOL. (USA), vol. 46, no. 5, 1983, pages 1125-1133, American Society for Microbiology, US; M.E. SANDERS et al.: "Characterization of phage-sensitive mutants from a phage-insensitive strain of Streptococcus lactis: evidence for a plasmid determinant that prevents phage adsorption"

APPLIED AND ENVIRONMENTAL MICROBI-OLOGY, vol. 47, no. 5, May 984, pages 979-985, Am. Soc. for Microbiology, US; M.E. Sanders et al.

(73) Proprietor: NORTH CAROLINA STATE UNIVER-SITY
103 Holladay Hall,
Campus Box 7003
Raleigh, North Carolina 27695-7003(US)

(72) Inventor: Klaenhammer, Todd Robert
6509 Bakersfield Drive
Raleigh North Carolina 27605(US)
Inventor: Steenson, Larry Ray
1300 Palmer Drive, Apartment 311
West Lafayette Indiana 47906(US)
Inventor: Sanozky-Dawes, Rosemary Barbara
2412 Cavalier Street
Raleigh North Carolina 27603(US)
Inventor: Sing, Wesley Davis
2435 Avent Ferry Road Apartment L
Raleigh North Carolina 27606(US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

APPLIED AND ENVIRONMENTAL MICROBI-OLOGY, vol. 49, no. 3, March 1985, pages 627-633, American Society for Microbiology, US; C.F. GONZALEZ et al.: "Transfer of su-crose-fermenting ability and nisin produc-tion phenotype among Lactic streptococci"

JOURNAL OF GENERAL MICROBIOLOGY, vol. 131, no. 6, June 1985, pages 1531-1542, SGM, GB; T.R. KLAENHAMMER et al.: "Conjugal transfer from Streptococcus lactis ME2 of plasmids encoding phage resistance, nisin resistance and lactose-fermenting ability: evidence for a high-frequency conjugative plasmid responsible for abortive infection of virulent bacteriophage"

CHEMICAL ABSTRACTS, vol. 105, no. 3, 21st July 1986, abstract no. 19507d, Columbus Ohio, US; W.M. DE VOS et al.: "Plasmid DNA in lactic streptococci: bacteriophage resis-tance and proteinase plasmids in Strepto-coccus cremorisSK11"

(74) Representative: **Bankes, Stephen Charles Dig-by et al**
**BARON & WARREN**
**18 South End**
**Kensington**
**London W8 5BU (GB)**

**Description**

The present invention relates to the plasmid pTR2030 and to derivatives thereof. More specifically, the present invention relates to the plasmid pTR2030 which carries one or more genetic determinants for phage resistance to group N streptococci and to derivatives of this plasmid which also carry at least one of the same genetic determinants.

Production of cheese and cultured dairy products has long relied on the fermentation of milk by group N streptococci. Members of this group, composed of Streptococcus lactis, S. cremoris, and S. lactis subsp. diacetylactis, are directly responsible for the acid development, flavor production, and often coagulum characteristics in mesophilic dairy fermentations. Because efficient milk fermentations are dependent on the growth and activity of the lactic streptococci, great care is exercised to prepare starter cultures that are highly active and uncontaminated with undesirable microorganisms or bacteriophages. However, the fermentation process itself is nonaseptic, occurring in open vats with a nonsterile medium, pasteurized milk. It is therefore highly susceptible to contamination with bacteriophages. For the majority of strains of lactic streptococci employed in commercial dairy fermentations, lytic bacteriophages capable of halting growth and acid production can appear within 1 to 2 days after introducing the culture into the cheese plant. Although bacteriophage contamination of numerous industrial fermentations has been observed, the destructive role of bacteriophages in milk fermentations is without parallel in other fermentation processes.

(Since the filing of this application, the taxonomic name of group N streptococci has been changed to "lactococci", the members of the group being redesignated as L. lactis, L. cremoris and L. lactis subsp. diacetylactis respectively.)

Historically, milk fermentations relied on starter cultures composed of undefined mixtures of lactic streptococci propagated without knowledge of, or protection from, bacteriophages. Natural phage contamination in these cultures established an equilibrium of evolving bacteriophages and phage-resistant variants. These cultures were highly variable in day-to-day levels of acid production, but remained moderately active and could be used continuously in small fermentation factories. Over the past 20 years, starter culture failures due to bacteriophage infection have become prevalent throughout the dairy industry. Increasing demand for cultured milk products in recent years has necessitated increases in both production capacity and process efficiency such that larger volumes of milk are processed, cheese vats are filled repeatedly within a single day, and total processing time is shortened. This modernization of the industry concurrently increased the probability of phage contamination and further dictated the use of defined mixtures of lactic streptococci capable of uniform and rapid rates of acid production. With the selection of highly fermentative lactic streptococci and their propagation under aseptic conditions (in the absence of bacteriophages), the majority of cultures now used by the industry have become highly susceptible to bacteriophage attack upon introduction into the cheese factory.

To cope with bacteriophage problems a number of successful methods have been developed to minimize phage action during commercial milk fermentations. Through the use of concentrated cultures, aseptic bulk starter vessels and phage-inhibitory media (see for example, U.S. Patent 4,282,255), the starter culture can be protected from bacteriophage infection prior to vat inoculation. However, phage contamination cannot be prevented following entrance into the fermentation vat. Therefore, emphasis for protection of the culture shifts to minimizing prolific phage-host interactions through rotation of phage-unrelated strains or use of phage-resistant mutants in multiple-strain starters. Although, in theory, strain rotation should minimize developing phage populations within the plant, in practice it has proved difficult to identify strains that demonstrate completely different patterns of phage sensitivity. Estimates of the total number of different, phage-unrelated lactic streptococci approximate 25 strains worldwide. Considering the small number of phage-unrelated strains available, the choice of strains for incorporation into rotation programs is severely limited. Similarly, few phage-unrelated strains are available for construction of multiple-strain starters containing composites of 4 to 6 strains.

A decade ago, Sandine, W.E. et al, J.Milk Food Technol. 35, 176 (1972) emphasized the need to isolate new strains of lactic streptococci for use in the dairy industry. Foremost among the criteria for selection of these strains was resistance to existing bacteriophages. It is now recognized that some strains of lactic streptococci are not attacked by any phage when challenged with large collections of laboratory phage banks, or when used on a continuous, long-term basis in commercial fermentations. These reports demonstrate the existence of lactic streptococci that are not sensitive to bacteriophage attack, in spite of devastating phage pressure such as that which routinely occurs within the factory environment. However, to date, only a limited number of phage-insensitive strains have been identified and studied for mechanisms of phage resistance.

3

Several mechanisms of phage resistance in group N streptococci have been identified and appear to be plasmid-associated. McKay, L.L. et al, Appl.Environ.Microbiol. 47, 68 (1984) describe a 40 megadalton plasmid, pNP40, found in S. lactis subsp. diacetylactis DRC3. When the plasmid was conjugally transferred to S. lactis C2, transconjugants were isolated which were resistant to phage c2 at 21°C and 32°C, but not at 37°C. It was found that the resistance to c2 was not due to an inhibition of phage adsorption or to a classical modification-restriction system, but was suggested to be a temperature-sensitive DNase. The authors concluded that the genetic determinant for this resistance was located on pNP40.

Sanders, M.E., et al, Appl.Environ.Microbiol. 47, 979 (1984) reported that S. lactis ME2 was insensitive to a variety of phages. The authors determined that this insensitivity was the result of several temperature-sensitive mechanisms including: (a) prevention of phage adsorption, (b) the modification-restriction system, and (c) suppression of phage development. The authors reported that the genetic determinants for some of the mechanisms may be found on plasmids, but that some appeared to be found on the chromosomes.

Sanders, M.E., et al, Appl.Environ.Microbiol. 46, 1125 (1983) disclose that S. lactis ME2 contains a plasmid, pME0030, which codes for a function that prevents phage adsorption. Sanders, M.E. et al, Appl.Environ.Microbiol. 42, 944 (1981) disclose that a 10 megadalton plasmid found in S. cremoris KH codes for a modification-restriction system which enables this strain to be resistant to phage c2.

Gonzalez, C.F. et al, Appl.Environ.Microbiol. 49, 627 (1985) reported that two transconjugants of two matings of S. lactis SLA 2.24 or SLA 3.15 and S. lactis subsp. diacetylactis SLA 3.10 or SLA 3.23, respectively, showed temperature-independent phage resistance which was not due to adsorption or restriction in phage growth. Physical evidence for plasmid involvement was not obtained.

The identification or creation of plasmids encoding phage resistance in group N streptococci is necessary in order to genetically engineer strains that meet industrial criteria for fermentative capabilities and long-term phage resistance. The present invention provides for a plasmid which confers phage resistance to group N streptococci. Group N streptococci containing the plasmid or a derivative thereof are useful for formulating starter cultures which can be used for the production of cheese and cultured dairy products.

The present invention comprises a plasmid or a derivative thereof which confers phage resistance to group N streptococci. The present invention also comprises group N streptococci containing the plasmid or derivative. The present invention further comprises starter cultures containing such streptococci.

More specifically, the present invention provides the plasmid pTR2030 characterized by a molecular weight of 30.0 ± 3.0 megadaltons, having the following sensitivity to restriction endonucleases:

| Enzyme | # sites |
|--------|---------|
| HindIII | 16 |
| HaeIII | 4 |
| EcoRI | 8 |
| XbaI | 5 |
| HpaI | 3 |
| NcoII | 5 |
| AvaI | 2 |

and carrying one or more genetic determinants for phage resistance in group N streptococci (lactococci) and exhibiting phenotypes of Tra+, Clu−, Hsp+ and Hrp+, the said plasmid being obtainable from S. Lactis TRSI-a(ATCC 53146) or S.Lactis TEK1 (ATCC 53167).

pTR2030 carries one or more genetic determinants which impart phage resistance. Derivatives of pTR2030 are considered herein to mean any plasmid capable of replication, transcription and translation in group N streptococci (lactococci) which carries the phage resistance genetic determinanets of pTR2030. The invention further comprises group N streptococci which contain the plasmid pTR2030 or derivatives thereof and starter cultures containing these group N streptococci. Preferred streptococci include strains of S.lactis, S. lactis subsp. diacetylactis and S. cremoris.

The present invention is directed to the plasmid pTR2030 and its derivatives which are useful for conferring phage resistance to group N streptococci. The latter are useful for formulating starter cultures for use in the production of cheese and cultured dairy products. pTR2030 and its derivatives could also be useful for conferring phage resistance to other gram-positive lactic acid bacteria used in dairy and food fermentation. pTR2030 carries one or more genetic determinants which confer phage resistance. The plasmid further carries genetic determinants for conjugal transfer. This genetic determinant promotes its own transfer as well as the transfer of other non-conjugative plasmids. A derivative of pTR2030 is used herein to refer to any plasmid capable of replication, transcription and translation in group N streptococci or other gram-positive lactic acid bacteria which is either (a) pTR2030 with inserted DNA sequences, or (b) pTR2030 with DNA sequences deleted, or (c) pTR2030 with inserted and deleted DNA sequences, or (d) a plasmid with a significant portion of at least one of the pTR2030 genetic determinant for phage resistance inserted therein.

Plasmid pTR2030 was isolated from a transconjugant of a mating of S. lactis ME2 and S. lactis LM0230. pTR2030 has a molecular weight of $30 \times 10^6$ daltons ± 10%. This corresponds to about 45 kilobases. pTR2030 arose as the result of the conjugal process and is distinct from plasmid pME0030, a 30 megadalton plasmid of S. lactis ME2 as evident from the available phenotypic evidence. First, it has been demonstrated that Hsp$^+$ (Sanders, M.E. et al, (1983), supra, and Sanders, M.E. et al (1984), supra) and Tra$^+$ are exhibited by S. lactis N1, a derivative of S. lactis ME2 which is cured of pME0030. pTR2030 encodes both Hsp$^+$ and Tra$^+$. Second, where the presence of pME0030 inhibits the adsorption of phage 18 to S. lactis ME2, the presence of pTR2030 in S. lactis LM0230 shows no effect on the adsorption of phage c2 or phage 18 (99% adsorption for both phages). Third, conjugation frequencies of Lac$^+$, Nis$^r$, and Hsp$^+$ occur at frequencies of $10^{-1}$/donor cell from transconjugants carrying pTR2030 as compared to frequencies of $10^{-6}$/donor cell from S. lactis ME2 carrying pME0030.

Table 1 shows the sensitivities of pTR2030 to restriction endonucleases. The sizes of the fragments produced are also shown in Table 1.

TABLE 1

| Restriction Enzyme | | | | | | | |
|---|---|---|---|---|---|---|---|
| | HindIII | HaeIII | EcoRI | XbaI | HpaI | NcoI | AvaI |
| # of fragments* | 16 | 4 | 8 | 5 | 3 | 5 | 2 |
| Sizes (Md) | 4.4 | 15.8 | 16.7 | 17.2 | 15.9 | 14.7 | 15.9 |
| | 3.0 | 3.9 | 7.8 | 5.3 | 12.1 | 13.0 | 14.4 |
| | 2.8 | 3.6 | 1.4 | 3.4 | 2.6 | 1.9 | |
| | 2.1 | 3.3 | 1.2 | 1.4 | | 1.2 | |
| | 2.0 | | 1.0 | 1.0 | | 0.8 | |
| | 1.9 | | 0.7 | | | | |
| | 1.6 | | 0.5 | | | | |
| | 1.3 | | 0.4 | | | | |
| | 1.2 | | | | | | |
| | 1.0 | | | | | | |
| | 0.9 | | | | | | |
| | 0.8~ | | | | | | |
| | 0.8~ | | | | | | |
| | 0.7 | | | | | | |
| | 0.6 | | | | | | |
| | 0.5 | | | | | | |
| Total (Md) | 25.6 | 26.6 | 29.7 | 28.3 | 30.6 | 31.6 | 30.3 |

* There may be small fragments undetected on the gels which were run under the following conditions: 0.8% ME agarose (FMC), 3 mm thick gel (submarine) in Tris-acetate buffer (40mM Tris, 12mM Na acetate, 1mM Na$_2$ EDTA, pH 7.8, with glacial acetic acid acid. Electrophoresis conditions at 5 volts/cm, 2.5 hours.

Plasmid pTR2030 exhibits phenotypes of Tra$^+$, Clu$^-$, Hsp$^+$, Hrp$^+$. These phenotypes are described as follows:

Tra$^+$: pTR2030 is a conjugative plasmid that promotes its own transfer and transfer of other non-conjugative plasmids. For example, pTR2030 is responsible for transfer of the non-conjugative plasmid pTR1040 (Lac$^+$, Nis$^r$). In the absence of pTR2030, conjugation of pTR1040 does not occur. Consequently, pTR2030 can be used for mobilizing other plasmids in group N streptococci and possibly other gram-positive bacteria (i.e., S. faecalis, lactobacilli).

Clu$^-$: pTR2030 exhibits high-frequency conjugal transfer in agar-surface matings. The frequency of conjugal transfer occurs at $10^{-1}$/donor cell in matings between S. lactis TRS1 (donor) and S. lactis LM2302 (recipient). pTR2030 does not induce the "clumping phenotype" (Clu[+]) in liquid media that has been observed previously for high-frequency conjugal plasmids of group N streptococci (Walsh, P.M. et al, J.Bacteriol. 146, 937 (1983); Anderson, D.G. et al, J.Bacteriol. 158, 954 (1984); Gasson, M.J. et al, J.Bacteriol. 143, 1260 (1980)). This Clu[−] phenotype is most desirable because the presence of this conjugal plasmid will not impose cell aggregation in transconjugants carrying pTR2030. Aggregating cultures are unsuitable for most applications in dairy and food fermentations.

Hsp[+]: pTR2030 exhibits a heat-sensitive phage resistance property when present in S. lactis LM0230. Cells containing pTR2030 that are challenged with c2 phage at 30°C form small plaques (~1 to 2 mm in diameter) at an efficiency of plaquing of 0.83. Normal plaque size for c2 phage on S. lactis LM0230 et 30°C is 4 to 5 mm in diameter. When cells containing pTR2030 are propagated and challenged with c2 phage et 40°C, normal plaque sizes of 4 to 5 mm are observed. Plaque size was correlated with the burst size of phage and found dependent on the presence of pTR2030 and the temperature of the assay. pTR2030-imposed reduction in burst and plaque size occurs at 30°C, but not at 40°C.

Hrp[+]: pTR2030 exhibits a heat-resistant phage resistance property when present in S. cremoris strains HP, M43a, 924, KH, and TDM1. Lytic phages virulent for the parent S. cremoris strains ($\phi$hp, $\phi$m12r•M12, $\phi$924, $\phi$kh, $\phi$18, respectively) show no plaque formation (efficiency of plaquing (EOP) < $10^{-9}$) on pTR2030 transconjugants under 30°C or 40°C incubation conditions. This Hrp[+] phenotype describes the temperature-independent restriction in plaquing efficiency for virulent phages on the S. cremoris transconjugants harboring pTR2030.

Derivatives of pTR2030 include the following plasmids: (a) plasmid pTR2030 into which DNA sequences have been inserted; (b) plasmid pTR2030 from which DNA sequences have been deleted; (c) plasmid pTR2030 into which DNA sequences have been inserted and from which DNA sequences have been deleted; and (d) any plasmid into which at least one of the pTR2030 genetic determinants for phage resistance has been inserted. Additionally, a derivative of pTR2030 can include any plasmid into which any DNA sequence of pTR2030 has been inserted. It is preferred that each of these derivatives contain at least one of the pTR2030 genetic determinants for phage resistance. It is further preferred that each of these derivatives contain the pTR2030 genetic determinants for conjugal transfer.

The derivatives of pTR2030 can be prepared by using techniques well known in the art. Thus, insertions and/or deletions to pTR2030 can be performed using standard techniques. Many standard techniques have been described by Maniatis, T. et al, Molecular Cloning: A Laboratory Manual, Cold Spring Harbor Laboratory (1982). Similarly, the pTR2030 genetic determinant for phage resistance (or any other pTR2030 DNA sequence) can be inserted into any plasmid using conventional techniques such as those described by Maniatis, T. et al, supra. A genetic determinant can be first isolated from pTR2030 and then inserted into a second plasmid. The second plasmid can be further tailored if necessary. A pTR2030 genetic determinant for phage resistance can be isolated as follows. Restriction fragments of pTR2030 are isolated and inserted into an appropriate plasmid vehicle for S. cremoris HP (ATCC No. 11602). The recombinant plasmid is then inserted into S. cremoris HP and the transformed bacteria analyzed for sensitivity to the phage $\phi$hp. Resistant colonies contain a plasmid carrying at least one of the pTR2030 genetic determinants for phage resistance. The restriction fragment with this determinant can be further manipulated as may be required, by using conventional techniques.

Suitable hosts for the plasmid pTR2030 or its derivetives are any microorganism in which pTR2030 or its derivatives are capable of replication. Preferred hosts are group N streptococci which include strains which are used in the production of cheese and cultured dairy products. Examples of these hosts are strains of S. lactis, S. lactis subsp. diacetylactis, and S. cremoris. Additional hosts include other gram-positive lactic acid bacteria which are used in dairy and food fermentations. Examples of these hosts include strains of Lactobacillus, Pediococcus and Leuconostoc. Plasmid pTR2030 or its derivatives can be introduced into any suitable host using standard techniques. Such techniques can include conjugal transfer as well as transformation.

Microorganisms containing pTR2030 or its derivatives preferably exhibit a phage resistance phenotype - As a result of this property, the microorganisms with pTR2030 or its derivatives are extremely useful in the production of cheese and cultured dairy products as well as other food and dairy fermentations. The microorganisms are capable of more efficient fermentation since they are not sensitive to phages which are present or likely to appear in the fermentation vessel. Microorganisms containing pTR2030 or its derivatives are useful in formulation of starter cultures for the production of cheese and cultured dairy products. The preparation and use of starter cultures are well known in the art. Consequently, starter cultures can be formulated by using a microorganism containing pTR2030 or its derivatives in place of a microorganism currently employed. For example, a strain of S. cremoris carrying pTR2030 can be used in preparing a starter culture in place of the original strain of S. cremoris lacking pTR2030.

The present invention will be further described by reference to the following non-limiting examples.

EXAMPLE 1

Isolation of Plasmid pTR2030

Streptococcus lactis ME2 (conjugal donor, Lac[+] Hsp[+] Nis[r] Str[s]) was mated with Streptococcus lactis LM0230 (plasmid-cured recipient, Lac[−] Hsp[−] Nis[s] Str[r]) on the surface of milk-glucose agar plates as described by McKay, L.L. et al, Appl.Environ.Microbiol. 40, 84 (1980). Donor and recipient cells were propagated for 4 hours et 30°C in M17-lactose or M17-glucose broths, respectively. M17 broth was used as described by Tarzaghi, B.E. et al, Appl. Microbiol. 29, 807 (1975). Lactose or glucose were present at 0.5% concentration. One part S. lactis ME2 cells was mixed with two parts S. lactis LM0230 cells and 0.2 ml of the suspension spread over the surface of a prepoured milk-glucose agar plate with a sterile glass rod. The plates were incubated for 18 hours at 30°C. Two ml of a broth containing 1% glucose and 5% non-fat dry milk solids were added to the surface of the plates and mixed with a sterile glass rod. One ml of this cell mixture was removed and 0.2 ml aliquots distributed over the surface of five selection plates containing lactose-indicator agar (McKay, L.L. et al, Appl. Microbiol. 23, 1090 (1972)) and 1 mg/ml streptomycin sulfate. The selection plates were incubated for 48 hours at 30°C and examined for the appearance of yellow colonies. Recombinants were confirmed by phenotype and genotype as Lac[+] Str[r] transconjugants. The frequency of Lac[+] transfer from the donor to the recipient by conjugation was 2.8 x $10^{-6}$/donor cell. All Lac[+] transconjugants were scored for nisin resistance or sensitivity initially in Elliker broth or Elliker agar (1.5% w/v agar, pH 6.5) containing 0.1% (v/v) Tween 20 and 100 ng/ml nisin. Nisin stock solutions were prepared in 0.02 M HCl as described by McKay, L.L. et al (1984), supra, and were diluted to the desired concentration in 10% (w/v) Elliker broth. All Lac[+] transconjugants were also Nis[r] and carried a 40 megadalton plasmid, pTR1040.

Lac[+] Nis[r] Str[r] transconjugants generated from matings between S. lactis ME2 and S. lactis LM0230 were examined for sensitivity to lytic phage c2. Plaque assays were conducted as follows. Phage adsorption was measured as described by Sanders, M.E., et al, Appl.Environ.Microbiol. 40, 500 (1980). Phage assay, i.e., PFU/ml. was measured as described by Terzaghi, B.E. et al, supra. Burst size was measured as follows. Cells (1 ml) from 3.5 h cultures propagated et 30°C or 40°C were added to a sterile Eppendorf microfuge tube (1.5 ml). After centrifugation for 3 minutes the supernate was discarded, the pellets resuspended in 1 ml fresh M17-glucose broth and 50 $\mu$l 1 M-CaCl$_2$•7H$_2$O and 100 $\mu$l c2 phage ($10^8$-$10^9$ p.f.u./ml) were added. The broth was mixed gently and incubated for 5 minutes et 30°C or 40°C to allow for adsorption of phage. After centrifugation for 4 minutes, the supernate was aspirated and the cells resuspended in 1 ml M17-glucose broth plus calcium (50$\mu$l 1 M-CaCl$_2$•7H$_2$O in 10 ml M17-glucose). This cell suspension was diluted to $10^{-4}$ in M17-glucose broth plus calcium. The diluted, phage-infected cell suspensions were incubated at 30°C or 40°C for 45 minutes. At 0 minutes and 45 minutes, 1 ml samples were removed and immediately subjected to plaque assay without treatment with chloroform. Burst size was calculated as the number of progeny phage et 45 minutes divided by the total number of infective centers at time 0. In such assays, 4% of the transconjugants exhibited plaques that were significantly smaller than those observed for phage c2 on the conjugal recipient, S. lactis LM0230. Burst size of c2 phage on the transconjugants at 30°C was reduced to 5 phage per cell, as compared to a burst size of 41 for c2 phage on the recipient, S. lactis LM0230. A 30 megadalton plasmid, designated pTR2030, was found to be responsible for the phage-resistance phenotype exhibited by these Lac[+] Nis[r] transconjugants selected from matings between S. lactis ME2 and S. lactis LM0230. One of these transconjugants, S. lactis TRS1, harbors both pTR1040 and pTR2030. Following growth of S. lactis TRS1 at 40°C, a Lac[−] Nis[s] derivative was isolated. This isolate exhibits Hsp[+], carries a 30 ± 3.0 megadalton plasmid (pTR2030), and was designated S. lactis TRS1-a (phenotype Hsp[+], Str[r],Lac[−]). TRS1-a containing pTR2030 was deposited on June 10, 1985 under the Budapest Treaty at the American Type Culture Collection, and has been assigned number 53,146.

Plasmid pTR2030 was isolated from S. lactis TRS1-a by the method of Klaenhammer, T.R., Current Microbiol. 10, 23 (1984) using the modifications described by Sanders, M.E. et al (1983), supra. Restriction endonucleases were obtained from Bethesda Research Laboratories and restriction digestions were performed using the method of Maniatis, T. et al, supra. Separation of DNA restriction fragments were accomplished on 0.8% agarose gels. Fragments generated by HindIII and EcoRI digestions of λ DNA served as moleculor weight standards. The results of restriction enzyme digestion are shown in Table 1, supra.

EXAMPLE 2

Preparation of S. lactis TEK1

For use in conjugation experiments to Str$^r$ recipients of S. cremoris a Str$^s$ donor strain which carried pTR1040 (Lac$^+$ Nis$^r$) and pTR2030(Hsp$^+$ Tra$^+$) was developed. Matings were conducted between S. lactis TRS1 and S. lactis C14$_5$ (plasmid-cured, Lac$^-$ Hsp$^-$ Str$^s$) as described above in Example 1. Lac$^+$ colonies were selected on lactose-indicator agar without added streptomycin. 100 Lac$^+$ colonies were examined for Hsp$^+$ and sensitivity to streptomycin. S. lactis TEK1 was detected as a Lac$^+$ Hsp$^+$ Str$^s$ transconjugant which carried pTR1040 and pTR2030. This culture was used as the donor of pTR2030 in conjugation experiments with S. cremoris Str$^r$ recipients as described further below. TEK1 containing pTR2030 and pTR1040 was deposited on June 25, 1985 under the Budapest Treaty at the American Type Culture Collection, and has been assigned number 53167.

EXAMPLE 3

Preparation of S. cremoris M43a

For use as conjugation recipients, Lac$^-$ variants of S. cremoris M12R were isolated on lactose-indicator agar following 24 hours of growth in M17-glucose broth containing 2 $\mu$g/ml ethidium bromide. Lac$^-$ variant M43 was purified by single colony isolation on lactose-indicator agar, propagated through M-l7 glucose broth and 0.1 ml spread over the surface of M17-glucose agar plates containing 250, 500, and 1,000 $\mu$g/ml streptomycin sulfate. After 24 to 48 hours at 30°C, single colonies appearing on plates with the highest concentration of streptomycin were picked and streaked onto M17-glucose agar containing 1 mg/ml streptomycin. One streptomycin-resistant (Str$^r$) variant was selected in this manner and designated M43a. The identity of the Lac$^-$ Str$^r$ variant was confirmed by plasmid composition and sensitivity to the S. cremoris M12R lytic phage (m12r-M12) in standard plaque assays as described in. Example 1. S. cremoris M43a was maintained in M17-glucose broth and 1 mg/ml streptomycin, but was propagated once in the absence of streptomycin before use in conjugation experiments.

EXAMPLE 4

Conjugal Transfer of pTR1040 and pTR2030

Matings were conducted between S. lactis ME2 as the donor strain and S. lactis LM0230 and S. cremoris M43a as the recipient strains, as described in Example 1. Lac$^+$ colonies were selected on lactose-indicator agar and 1 mg/ml streptomycin sulfate as described in Example 1. Nisin resistance was examined as described in Example 1, except that 50 ng/ml of nisin was used instead of 100 ng/ml. Phage resistance was determined by the procedure described in Example 1. Phage resistance was dictated by (a) a reduction in plaque and burst sizes for c2 phage on S. lactis LM0230 Lac$^+$ transconjugants, and (b) inability of m12r•M12 phage to form plaques on S. cremoris M43a Lac$^+$ transconjugants. The results of the conjugation experiment are shown in Table 2. Samples of either ME2 or LM0230 alone gave no recombinants. Frequency was less than 7.6 x 10$^{-9}$ recombinants/donor.

TABLE 2

| Recipient | Lac$^+$ Str$^r$ Recombinants | | | |
|---|---|---|---|---|
| | per ml | frequency/donor | % nisin resistant | % phage resistant |
| S. lactis LM0230 | 369 | 2.8 x 10$^{-6}$ | 100 | 4 |
| S. cremoris M43a | 19,010 | 1.1 x 10$^{-4}$ | 100 | 57 |

8

EXAMPLE 5

Hsp$^+$ and Hrp$^+$ Phenotypes in pTR2030 Transconjugants

One of the transconjugants of S. lactis LM0230 and S. lactis ME2 produced in Example 1 was sensitive to phage c2. This transconjugant was identified as S. lactis TRS3 and was found to contain only plasmid pTR1040 and not pTR2030. Matings were conducted between S. lactis ME2 and S. cremoris M43a and examined as described in Example 1. One of the transconjugants was Lac$^+$ Nis$^r$, which was phage resistant and was found to contain both pTR1040 and pTR2030. This transconjugant was identified as S. cremoris T2r-43a. A second transconjugant which was phage sensitive was found to contain only pTR1040 and not pTR2030. This second transconjugant was identified as S. cremoris T2s-M43a. The adsorption, burst size and efficiency of plaquing (EOP) of phage on S. lactis strains LM0230, TRS1 and TRS3, and on S. cremoris strains M43a, T2s-M43a and T2r-M43a were determined using phage c2 and m12r•M12, respectively, as described in Example 1. The results are shown in Table 3.

TABLE 3

| Strain | Plasmids | Phenotype | % adsorption Phage | EOP | Burst Size at | |
|---|---|---|---|---|---|---|
| | | | | | 30°C | 40°C |
| S. lactis | | | | | | |
| LM0230 | -- | Lac$^-$Nis$^s$Hsp$^-$ | 99% | 1.0 | 40 | 56 |
| TRS3 | pTR1040 | Lac$^+$Nis$^r$Hsp$^-$ | 96% | 1.0 | 41 | 66 |
| TRS1 | pTR1040 pTR2030 | Lac$^+$Nis$^r$Hsp$^+$ | 98% | 0.83 | 5 | 51 |
| S. cremoris | | | | | | |
| M43a | -- | Lac$^-$Nis$^s$Hrp$^-$ | 99% | 1.0 | NA | NA |
| T2s-M43a | pTR1040 | Lac$^+$Nis$^r$HrP$^-$ | ND | 1.1 | NA | NA |
| T2r-M43a | pTR1040 pTR2030 | Lac$^+$Nis$^r$Hrp$^+$ | 98% | <4.3 x 10$^{-10}$ | NA | NA |
| NA = not applicable ND = not determined | | | | | | |

EXAMPLE 6

Demonstration of Tra$^+$ of pTR2030

Matings were conducted between various donor strains identified in Table 4 and a recipient strain S. lactis LM2302 (Lac$^-$ Nis$^s$ Ery$^r$ Str$^r$), and transconjugants were selected on lactose-indicator agar containing 15 ug/ml erythromycin and 1 mg/ml streptocycin as described in Example 1. The results shown in Table 4 demonstrate that pTR2030 promotes high-frequency transfer of Hsp$^+$ and Hrp$^+$ (found on pTR2030) and the non-conjugative plasmid pTR1040 encoding Lac$^+$ and Nis$^r$.

TABLE 4

| Donor | Plasmids | Phenotype of Donor | $Lac^+Nis^rEry^rStr^r$ recombinants | | |
|---|---|---|---|---|---|
| | | | per ml | freq./donor | %$Hsp^+$/$Hrp^+$ |
| TRS1 | pTR1040 pTR2030 | $Lac^+Nis^rHsp^+$ | $9.6 \times 10^6$ | $1.2 \times 10^{-1}$ | 85% |
| TRS3 | pTR1040 | $Lac^+Nis^rHsp^-$ | 0 | $<3.8 \times 10^{-10}$ | 0% |
| T2r-M43a | pTR1040 pTR2030 | $Lac^+Nis^rHrp^+$ | $1.7 \times 10^6$ | $2.6 \times 10^{-2}$ | 67% |
| T2s-M43a | pTR1040 | $Lac^+Nis^rHrp^-$ | 0 | $<5.2 \times 10^{-8}$ | 0% |

EXAMPLE 7

Conjugal transfer of pTR2030 to strains of S. cremoris

Matings were conducted between donor strains ($Lac^+$ $Nis^r$ $Str^-$ $Tra^+$ $Hsp^+$) and recipient strains ($Lac^-$ $Nis^s$ $Str^r$ of S. cremoris as shown in Table 5, and transconjugants were selected as described in Example 1. Phage-resistant transconjugants were $Hrp^+$ and confirmed to carry pTR2030. The results are shown in Table 5. It can be seen that pTR2030 can be conjugally transferred between these strains.

TABLE 5

| Mating Pair | | $Lac^+$ $Str^r$ Transconjugants | |
|---|---|---|---|
| Donor S. lactis | Recipient S. cremoris | Freq./Donor | % Phage Resistant ($Hrp^+$) |
| TEK1 | M43a | $3.3 \times 10^{-2}$ | 27% |
| TEK1 | KHA2 | $4.5 \times 10^{-6}$ | 25% |
| TEK1 | HPA4 | $4.0 \times 10^{-4}$ | 20% |
| TEK1 | 924EB1 | $1.1 \times 10^{-4}$ | 28% |
| ME2 | TDM1R3 | $6.9 \times 10^{-5}$ | 62% |

EXAMPLE 8

Effect of Phage on S. cremoris Transconjugants

Plaque assays with various S. cremoris strains were determined as described in Example 1. The results are shown in Table 6. It can be seen that S. cremoris strains containing pTR2030 were resistant to the phage tested, i.e., the phages failed to replicate on those strains.

TABLE 6

| Strain | Description | Phage | PFU/ml* | EOP |
|---|---|---|---|---|
| M43a | phage sensitive parent | m12r•M12 | $1.4 \times 10^{10}$ | 1.0 |
| TM43a | pTR2030 transconjugant | m12r•M12 | <10 | $<7.1 \times 10^{-10}$ |
| KH | phage sensitive parent | $\phi$kh | $5.6 \times 10^9$ | 1.0 |
| TKH1 | pTR2030 transconjugant | $\phi$kh | <10 | $<1.8 \times 10^{-9}$ |
| HP | phage sensitive parent | $\phi$hp | $6.7 \times 10^9$ | 1.0 |
| THP14 | pTR2030 transconjugant | $\phi$hp | <10 | $<1.5 \times 10^{-9}$ |
| 924 | phage sensitive parent | $\phi$924 | $1.9 \times 10^9$ | 1.0 |
| T9249 | pTR2030 transconjugant | $\phi$924 | <10 | $<5.3 \times 10^{-9}$ |
| TDM1 | phage sensitive parent | $\phi$18 | $3.6 \times 10^{10}$ | 1.0 |
| TM-TDM1R3 | pTR2030 transconjugant | $\phi$18 | <10 | $<2.8 \times 10^{-1}$ |

* Plaque forming units/ml of phage suspension

## EXAMPLE 9

### Effect of Phages on S. cremoris M12 and M43a

Virulent phages were independently isolated from cheese plants using a starter culture comprising S. cremoris M12 cells. Plaque essays with S. cremoris M12 and S. cremoris T2r-43a were determined as described in Example 1. The results are shown in Table 7, where it can be seen that the transconjugant containing pTR2030, i. e., S. cremoris T2r-M43a, was resistant to all phages tested (i.e., the phages failed to replicate on this strain).

TABLE 7

| Phage | M12 | | T2r-M43a | |
|---|---|---|---|---|
| | Titer, PFU/Ml | EOP | Titer, PFU/ml | EOP |
| m12r•M12 | $2.8 \times 10^{10}$ | 1.0 | <10 | $<3.6 \times 10^{-10}$ |
| $\phi$05 | $3.8 \times 10^{10}$ | 1.0 | <10 | $<2.7 \times 10^{-10}$ |
| da | $4.6 \times 10^9$ | 1.0 | <10 | $<2.2 \times 10^{-9}$ |
| br | $5.8 \times 10^9$ | 1.0 | <10 | $<1.7 \times 10^{-9}$ |
| ot | $1.9 \times 10^{10}$ | 1.0 | <10 | $<5.3 \times 10^{-10}$ |
| sgl | $3.2 \times 10^{10}$ | 1.0 | <10 | $<3.1 \times 10^{-10}$ |

## EXAMPLE 10

### Starter Culture Activity Test

The starter culture activity test was a modification of that described by Heap, H.A. et al, N.Z.J. Dairy Sci.Technol. 11, 16 (1976). Cultures were prepared by overnight incubation at 30°C in 11% reconstituted skim milk (RSM, steamed for one hour); or in 11% RSM containing 0.25% casamino acids (RSM/CA) for growth of proteinase-negative strains (for M43a and T2r-M43a only). Phage suspension (200 $\mu$l, ~$10^8$ PFU/ml) was mixed with 200 $\mu$l of milk culture and incubated at room temperature for 10 minutes to allow adsorption of phage. RSM or RSM/CA (9.6 ml) containing 40 $\mu$g/ml bromocresol purple (BCP) was added and- the tubes were sequentially incubated for 180 minutes at 30°C, 190 minutes at 40°C, and 150 minutes at 30°C for S. cremoris M43 and T2r-M43a, and 100 minutes at 30°C, 190 minutes at 40°C and 100 minutes at 30°C for all other strains. Following the incubation, milk samples were examined for pH and phage titer. The initial pH of the milk was 6.5 - 6.6. The results given in Table 8 show that virulent phages failed to replicate on strains of S. cremoris which contained pTR2030 and did not inhibit acid development during the starter culture activity test.

**TABLE 8**

| Strain | Description | Phage | Phage Titer (PFU/ml) | | Final pH |
|---|---|---|---|---|---|
| | | | Initial | Final | |
| S. cremoris M43 | Lac$^+$ parent | -- | -- | -- | 5.9 |
| | | +m12r·M12 | $4.1 \times 10^6$ | $5.2 \times 10^9$ | 6.5 |
| S. cremoris T2r-M43a | pTR2030 transconjugant | -- | -- | -- | 5.9 |
| | | +m12r·M12 | $4.1 \times 10^6$ | $2.7 \times 10^3$ | 5.9 |
| S. cremoris KH | Lac$^+$ parent | -- | -- | -- | 5.6 |
| | | +φkh | $3.0 \times 10^6$ | $1.3 \times 10^{10}$ | 6.4 |
| S. cremoris TKH1 | pTR2030 transconjugant | -- | -- | -- | 5.4 |
| | | +φkh | $3.0 \times 10^6$ | $4.0 \times 10^3$ | 5.4 |
| S. cremoris HP | Lac$^+$ parent | -- | -- | -- | 5.8 |
| | | +φhp | $3.1 \times 10^6$ | $4.9 \times 10^9$ | 6.4 |
| S. cremoris THP14 | pTR2030 transconjugant | -- | -- | -- | 5.8 |
| | | +φhp | $3.1 \times 10^6$ | $1.9 \times 10^3$ | 5.9 |
| S. cremoris 924 | Lac$^+$ parent | -- | -- | -- | 5.7 |
| | | +φ924 | $6.7 \times 10^5$ | $1.8 \times 10^8$ | 6.0 |
| S. cremoris T9249 | pTR2030 transconjugant | -- | -- | -- | 5.8 |
| | | +φ924 | $6.7 \times 10^5$ | $9.5 \times 10^4$ | 5.8 |
| S. cremoris TDM1 | Lac$^+$ parent | -- | -- | -- | 4.9 |
| | | +φ18 | $5.2 \times 10^6$ | $3.0 \times 10^9$ | 6.4 |
| S. cremoris TM-TDM1R3 | pTR2030 transconjugant | -- | -- | -- | 5.1 |
| | | +φ18 | $5.2 \times 10^6$ | $7.0 \times 10^6$ | 5.1 |

A consecutive starter culture activity test was conducted using m12·M12 phage to determine whether or not virulent phage could be generated which was active against the S. cremoris T2r-M43a transconjugant. Repeated cycles of the activity test were conducted as above except that 100 μl of m12r-M12 phage suspension (~$10^8$ PFU/ml, prepared in M17 broth) and 100 μl of whey collected from the previous activity test were mixed with 200 μl of cells prior to milk inoculation. It was found that virulent phage also failed to develop toward S. cremoris T2r-M43a which contained pTR2030 in the consecutive starter culture activity test.

While the invention has been described in connection with specific embodiments thereof, it will be understood that it is capable of further modifications. This application is intended to cover any variations, uses or adaptations of the invention following, in general, the principles of the invention and including such departures from the present disclosure as come within known and customary practice within the art to which the invention pertains.

## Claims

1. The plasmid pTR2030 characterized by a molecular weight of 30.0 ± 3.0 megadaltons, having the following sensitivity to restriction endonucleases:

| Enzyme | # Sites |
|--------|---------|
| HindIII | 16 |
| HaeIII | 4 |
| EcoRI | 8 |
| XbaI | 5 |
| HpaI | 3 |
| NcoII | 5 |
| AvaI | 2 |

and carrying one or more genetic determinants for phage resistance in group N streptococci (lactococci) and exhibiting phenotypes of Tra$^+$, Clu$^-$, Hsp$^+$ and Hrp$^+$, the said plasmid being obtainable from S. Lactis TRSI-a(ATCC 53146) or S.Lactis TEK1 (ATCC 53167).

2. A plasmid which comprises a derivative of the plasmid pTR2030 of claim 1 wherein said derivative carries at least one of said genetic determinants for phage resistance.

3. A plasmid according to claim 2 wherein said derivative is selected from:
   (a) the plasmid pTR2030 into which one or more DNA segments have been inserted;
   (b) the plasmid pTR2030 from which one or more DNA segments have been deleted;
   (c) the plasmid pTR2030 into which one or more DNA segments have been inserted and from which one or more DNA segments have been deleted, and
   (d) any plasmid into which at least one of said genetic determinants for phage resistance from the plasmid pTR2030 has been inserted.

4. A plasmid according to claim 2 or claim 3 wherein said derivative further carries genetic determinants of plasmid pTR2030 for conjugal transfer.

5. A group N streptococcus (lactococcus) containing a plasmid according to any preceding claim.

6. A group N stretptococcus according to claim 5 which is selected from S.lactis, S. lactis subsp. diacetylactis and S.cremoris.

7. A starter culture for fermenting milk which comprises a group N Streptococcus according to claim 6.

8. A starter culture for fermenting food which comprises a group N Streptococcus according to claim 5.

## Patentansprüche

1. Plasmid pTR2030, gekennzeichnet durch ein Molekularge-wicht von 30,0 ± 3,0 Megadalton, daß die folgende Empfindlichkeit gegen Restriktionsendonucleasen aufweist:

| Enzym | Anzahl/Stellen |
|--------|----------------|
| HindIII | 16 |
| HaeIII | 4 |
| EcoRI | 8 |
| XbaI | 5 |
| HpaI | 3 |
| NcoII | 5 |
| AvaI | 2 |

und eine oder mehrere genetische Determinanten für die Phagen-Resistenz von Streptokokken der Gruppe N (Lactokokken) trägt und die Phänotypen Tra$^+$, Clu$^-$, Hsp$^+$ und Hrp$^+$ zeigt, wobei das Plasmid aus S. Lactis TRSI-a(ATCC 53146) oder S. Lactis TEK1 (ATCC 53167) erhältlich ist.

2. Plasmid, das ein Derivat des Plasmids pTR2030 nach Anspruch 1 enthält, wobei das Derivat mindestens eine der genetischen Determinanten für die Phagen-Resistenz trägt.

3. Plasmid nach Anspruch 2, wobei das Derivat ausgewählt ist aus:
   (a) dem Plasmid pTR2030, in das ein oder mehrere DNA-Segmente insertiert wurden;
   (b) dem Plasmid pTR2030, aus dem ein oder mehrere DNA-Segmente deletiert wurden;
   (c) dem Plasmid pTR2030, in das ein oder mehrere DNA-Segmente insertiert wurden und aus dem ein oder mehrere DNA-Segmente deletiert wurden, und
   (d) jedem Plasmid, in das mindestens eine der genetischen Determinanten für die Phagen-Resistenz aus dem Plasmid pTR2030 insertiert wurde.

4. Plasmid nach Anspruch 2 oder Anspruch 3, wobei das Derivat außerdem genetische Determinanten des Plasmids pTR2030 zur Transferierung durch Konjugation trägt.

5. Streptokokkus der Gruppe N (Lactokokkus), der ein Plasmid nach einem der vorhergehenden Ansprüche enthält.

6. Streptokokkus der Gruppe N nach Anspruch 5, der aus S. Lactis, S. Lactis-Unterart Diacetylactis und S. Cremoris ausgewählt ist.

7. Starter-Kultur zur Milchfermentierung, die einen Streptokokkus der Gruppe N nach Anspruch 6 enthält.

8. Starter-Kultur zur Lebensmittelfermentierung, die einen Streptokokkus der Gruppe N nach Anspruch 5 enthält.

**Revendications**

1. Plasmide pTR2030 caractérisé par une masse moléculaire de 30,0 ± 3,0 mégadaltons, ayant la sensibilité aux endonucléases de restriction suivante :

EP 0 208 468 B1

| Enzyme | # sites |
|--------|---------|
| HindIII | 16 |
| HaeIII | 4 |
| EcoRI | 8 |
| XbaI | 5 |
| HpaI | 3 |
| NcoII | 5 |
| AvaI | 2 |

et portant un ou plusieurs déterminants génétiques de la résistance au phage dans le groupe de streptocoques N (lactocoques) et présentant des phénotypes de $Tra^+$, $Clu^-$, $Hsp^+$ ainsi que de $Hrp^+$, ledit plasmide pouvant être obtenu à partir de S. Lactis TRSI-a(ATCC 53146) ou S.Lactis TEK1 (ATCC 53167).

2. Plasmide qui comprend un dérivé de plasmide pTR2030 selon la revendication 1, dans lequel ledit dérivé porte au moins un desdits déterminants génétiques de la résistance au phage.

3. Plasmide selon la revendication 2, dans lequel ledit dérivé est choisi parmi :
    (a) le plasmide pTR2030 dans lequel on a inséré une ou plusieurs séquences d'ADN ;
    (b) le plasmide pTR2030 duquel on a enlevé une ou plusieurs séquences d'ADN ;
    (c) le plasmide pTR2030 dans lequel on a inséré une ou plusieurs séquences d'ADN et duquel on a enlevé une ou plusieurs séquences d'ADN, et
    (d) un plasmide quelconque dans lequel on a inséré au moins un desdits déterminants génétiques de la résistance au phage provenant du plasmide pTR2030.

4. Plasmide selon la revendication 2 ou 3 dans lequel ledit dérivé porte en outre des déterminants génétiques du plasmide pTR2030 pour un transfert conjugal.

5. Groupe de Streptococcus N (lactococcus) qui comprend un plasmide selon l'une quelconque des revendications précédentes.

6. Groupe de streptococcus N selon la revendication 5 qui est choisi parmi S.lactis, S.Lactis sous-espèce diacetylactis et S.cremoris.

7. Levain pour la fermentation du lait qui comprend un groupe de Streptococcus N selon la revendication 6.

8. Levain pour la fermentation d'aliments qui comprend un groupe de Steptococcus N selon la revendication 5.

15